# EUROPEAN PATENT SPECIFICATION

(11) **EP 3 285 700 B1**
(45) Date of publication and mention of the grant of the patent: **14.04.2021**
(21) Application number: 16783843.2
(22) Date of filing: 21.04.2016
(51) Int. Cl.: A61F 2/966

(54) **SYSTEMS FOR STENT DELIVERY**
SYSTEME ZUR STENTEINFÜHRUNG
SYSTÈMES PERMETTANT LA POSE D'UNE ENDOPROTHÈSE

(30) Priority: 21.04.2015 US 201562150638 P
(43) Date of publication of application: 28.02.2018
(73) Proprietor: Boston Scientific Scimed, Inc., Maple Grove, MN 55311 (US)
(72) Inventor: NEGLEN, Peter, Saint Louis, Missouri 63131 (US); KAO, Stephen, Saint Louis, Missouri 63131 (US); CHINUBHAI, Abha, Saint Louis, Missouri 63131 (US); LAGOE, Daniel, Saint Louis, Missouri 63131 (US); TRAN, Lieu, Saint Louis, Missouri 63131 (US)
(74) Representative: Vossius & Partner Patentanwälte Rechtsanwälte mbB
(86) International application number: PCT/US2016/028613
(87) International publication number: WO 2016/172324

(56) References cited:
- WO-A1-2012/058580
- US-A- 5 201 757
- US-A- 5 980 533
- US-A1- 2011 301 685
- US-A1- 2012 296 407

## Description

### CROSS-REFERENCE TO RELATED APPLICATIONS

### BACKGROUND

Endoluminal delivery systems for prostheses such as stents, or other implantable structures may be used for placement of a stent in the arterial system, the venous system, or any other portion of the body. The use of stents and other implantable medical devices such as grafts, stent-grafts, filters, shunts, valves, etc., are referred to herein as prostheses. Prostheses may be used to deliver drugs to tissue, support tissue, or maintain patency of bodily lumens, as well as performing other functions, and have been widely reported in the scientific and patent literature.

Stents are typically delivered via a catheter in an unexpanded configuration to a desired location in the body. The combined stent and catheter is may be referred to as the stent delivery system. Once at the desired location, the stent is expanded and implanted into the body lumen. Examples of locations in the body include, but are not limited to, arteries (e.g. aorta, coronary, carotid, cranial, iliac, femoral, etc.), veins (e.g. vena cava, jugular, iliac, femoral, hepatic, subclavian, brachiocephalic, azygous, cranial, etc.), as well as other locations including the esophagus, biliary duct, trachea, bronchials, duodenum, colon, and ureter.

Typically, a stent will have an unexpanded configuration with reduced diameter for placement and an expanded configuration with expanded diameter after placement in the vessel, duct, or tract. Some stents are self-expanding, and some stents are mechanically expanded with a radial outward force applied from within the stent (e.g. with a balloon). Some stents have one or more characteristics common to both self-expanding and mechanically expandable stents.

In many stent delivery systems, particularly those used to deliver a self-expanding stent, the stent is typically retained on the catheter in its unexpanded form with a constraining member or other retention device such as a sheath or outer shaft. Precise delivery of venous stents can be challenging, while accurate placement of the stent implant is desirable in order to achieve optimal clinical outcomes.

Conventional stent delivery systems deploy the stent through the distal most opening of the delivery system, in a distal to proximal direction relative to the catheter. The physician is able to reposition the leading end of the stent prior to full stent deployment. This conventional positioning allows the physician to achieve placement accuracy of the leading end of the stent. However, due to stent foreshortening and variability in vessel diameter along the length of the vessel, conventional systems do not allow for accurately predicting the final positioning of the trailing end of the stent until the stent is fully deployed. Accordingly, the trailing end may not be accurately or optimally positioned US 2011/301685 A1 discloses a stent delivery system allowing the prosthesis to radially expand from a proximal end thereof to a distal end thereof.

In many instances, however, the physician will require accurate placement of the proximal end of the stent that cannot be achieved using a conventional delivery system. For example, accurate placement is often necessary to prevent unintentional jailing or blocking of major side branches/tributaries and to ensure full coverage of the targeted treatment zone within the diseased vasculature. In both cases, additional intervention would be required by the physician if accurate stent placement was not achieved at the trailing end of the stent.

Accordingly, it would be desirable to provide improved stent delivery systems that can accurately deliver a prosthesis such as a stent to a desired treatment site, including providing an accurate final positioning of the trailing end of the stent (e.g., proximal end of the stent implant). In certain parts of the anatomy, exact placement of the stent is critical to the successful clinical outcome of the procedure. For example, precise deployment is desirable with ilio-femoral stenting as is routinely used for the treatment of iliac vein compression syndrome (IVCS) and post-thrombotic syndrome (PTS) whereby the profunda and the common femoral vein can be partially or completely blocked (or "stent jailed") by the stent if the stent is not placed accurately after deployment.

In situations where multiple stents are delivered, it may be desirable to selectively deploy the stents. For example, abdominal aortic aneurysm (AAA) stent-grafts can be constructed of multiple components - trunk or main body, bifurcated main, main extension, limb extensions, stepped limbs, flared limbs, etc. Because each component is placed and deployed with a preferred release, a combination of proximal or distal releases may be desirable. This type of stenting can be useful in other areas of the body where bifurcations are present as well.

Therefore, it would be desirable to deploy a stent from its proximal end toward its distal end instead of from the distal end toward its proximal end, as is traditionally done in conventional stent procedures.

### SUMMARY

In one or more embodiments according to the invention, a stent delivery system is provided that includes an inner elongate shaft having a proximal portion and a distal portion, and a radially expandable prosthesis disposed over the inner elongate shaft. The prosthesis has a radially collapsed configuration and a radially expanded configuration, wherein in the collapsed configuration the prosthesis is adapted to be delivered through a vasculature, and in the expanded configuration the prosthesis engages a vessel wall. The stent delivery system further includes an outer elongate shaft having a proximal portion and a distal portion and a sheath or cover having a proximal portion and a distal portion, the sheath or cover disposed over the radially expandable prosthesis. A first hub is coupled to the outer shaft and a second hub is disposed proximally of the first hub. A hypotube is coupled to the second hub and extends distally toward the first hub. The distal advancement of the inner shaft advances the sheath or cover distally, thereby allowing the prosthesis to radially expand from a proximal end thereof to a distal end thereof.

In one or more embodiments, a system is provided that includes a delivery subsystem having a nose tip coupled with a sheath such that the nose tip and sheath are movable together, wherein the sheath is configured to receive therein a stent for deployment. The stent surrounds an inner shaft of the delivery subsystem. The system further includes an actuating subsystem configured to advance the sheath along the inner shaft from a proximal to distal direction to deliver the stent in a reverse deploy direction.

In one or more examples (not forming part of the invention), a method for deploying a prosthesis is provided. The method includes providing a delivery catheter comprising a prosthesis having a proximal end and a distal end, wherein the prosthesis is in a collapsed configuration and disposed on the delivery catheter. The method further includes delivering the prosthesis to a target site, wherein the deployment direction comprises radially expanding the prosthesis from the proximal end thereof to the distal end thereof. The method also includes removing a constraint from the prosthesis thereby permitting the prosthesis to radially expand in the peripheral deployment direction. The method additionally includes radially expanding the prosthesis from the collapsed configuration to an expanded configuration in the peripheral deployment direction so that the expanded prosthesis engages tissue at the target site, and withdrawing the delivery catheter from the patient and leaving the prosthesis deployed at the target site

### BRIEF DESCRIPTION OF THE DRAWINGS

Figure 1 is a diagram illustrating a stent delivery system in various stages or states of deployment.
Figure 2 is diagram of the stent delivery system of Figure 1 showing various components in a non-deployed stage or state.
Figure 3 is diagram of the stent delivery system of Figure 1 showing various components in a partially deployed stage or state.
Figure 4 is diagram of the stent delivery system of Figure 1 showing various components in a fully deployed stage or state.
Figure 5 is a diagram illustrating delivery of a stent using stent delivery systems.
Figure 6 is another diagram of a stent delivery system.
Figure 7 is a diagram of a packaging arrangement for a stent delivery system.
Figure 8 is a diagram of a process for stent deployment.

### DETAILED DESCRIPTION OF THE EMBODIMENTS

The following detailed description of certain embodiments will be better understood when read in conjunction with the appended drawings. To the extent that the figures illustrate diagrams of the hardware or functional blocks of various embodiments, the blocks are not necessarily indicative of the division between various components. Thus, for example, one or more of the functional blocks may be implemented in a single piece of hardware or multiple pieces of hardware. Moreover, one or more blocks or embodiments may be used together or separately.

As used herein, the terms "system," "subsystem," "unit," or "module" may include any combination of hardware that is operable or configured to perform one or more functions.

As used herein, an element or step recited in the singular and proceeded with the word "a" or "an" should be understood as not excluding plural of said elements or steps, unless such exclusion is explicitly stated. Furthermore, references to "one embodiment" are not intended to be interpreted as excluding the existence of additional embodiments that also incorporate the recited features. Moreover, unless explicitly stated to the contrary, embodiments "comprising" or "having" an element or a plurality of elements having a particular property may include additional such elements not having that property.

Various embodiments provide systems and methods for stent delivery, particularly to deploying a stent in a direction reverse to a conventional deployment. For example, conventional stent delivery systems deploy the stent in a distal to proximal direction (relative to the delivery system). This system allows for repositioning of the distal end of the stent prior to full deployment. As a result, placement accuracy of only the distal end of the stent implant can be achieved.

In contrast, and advantageously, various embodiments provide a "peripheral deploy" or "reverse deploy" or "proximal deploy" stent delivery system that is designed to deploy the stent implant in a proximal to distal direction relative to the delivery system. In one or more embodiments, the system is configured as a "peripheral only deploy" or "reverse only deploy" or "proximal only deploy" stent delivery system that is designed to deploy the stent implant only in a proximal to distal direction relative to the delivery system (i.e., not in a distal to proximal direction). However, variations and modifications are contemplated in other embodiments, such as to provide different directions of deployment or operation with different systems having different directions of deployment.

Various embodiments provide the technical effect of allowing for positioning and/or repositioning of the proximal end of the stent prior to full deployment. As a result, various embodiments provide improved placement accuracy of the proximal end of the stent implant as compared to the conventional stent delivery systems discussed above. This improved placement accuracy of the proximal end of the stent (relative to the delivery system) also helps ensure full coverage of the diseased vessel and reduces or minimizes the risk of jailing major tributaries.

By using one or more embodiments or practicing one or more embodiments, accurate placement of a peripheral stent may be provided with a reduced cost delivery system. The delivery system may be manufactured, assembled and/or inspected in a more streamlined process. Additionally, improved material sourcing may be provided.

It should be noted that although various embodiments may be described in connection with a particular stent or placement in a particular location, the various embodiments may be implemented for use with different types of stents for placement in different locations.

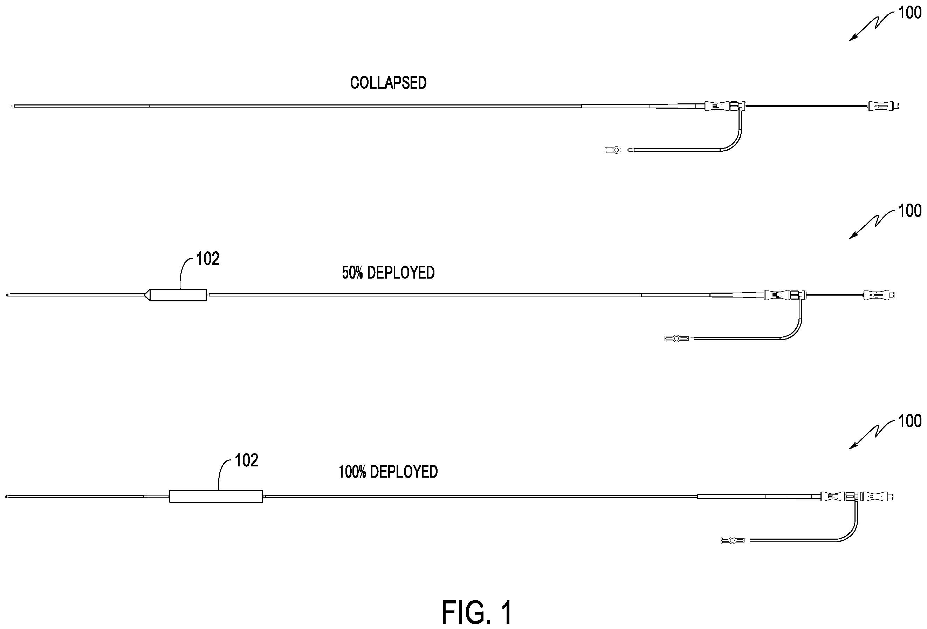

Various embodiments provide a stent delivery system 100 as shown in Figures 1-6, which is configured as a peripheral deploy stent system or a reverse deploy stent system. For example, in various embodiments, the stent delivery system 100 is configured to provide for deployment of a stent 102 (or radially expandable prosthesis) in a reverse direction (also referred to as a bottom to top direction or a proximal to distal direction).

The stent delivery system 100 in some embodiments may include as the stent 102, an implantable stent and a stent delivery system (or subsystem). In one embodiment, the stent 102 is a laser cut self-expanding stent formed of nickel titanium alloy (nitinol) with closed-cell segments and flexible interconnections designed specifically for use in venous anatomy. One example of a stent that may be used with one or more embodiments is the Vici stent implant available from Veniti of St. Louis, Missouri. For example, the stent delivery system 100 may be used in combination with a stent as described in U.S. Patent No. 9,233,014.

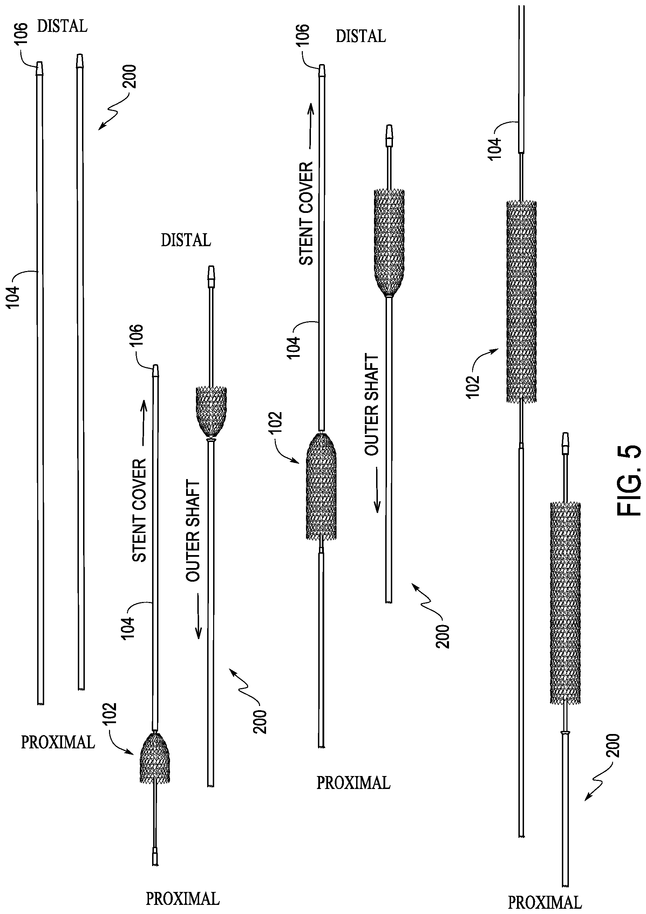

In the illustrated embodiments, the stent delivery system 100 has a coaxial design with an outer stent cover 104 (or sheath) to protect and constrain the stent 102. During delivery, the outer stent cover 104 is pushed distally (relative to the stent delivery system 100) to expose and deploy the stent 102 in a peripheral to distal direction, which allows for more accurate placement of the stent, particularly of the peripheral end thereof. The deployment direction provided by the stent delivery system 100 is different than in conventional deploy systems as illustrated in Figure 5, showing the stent delivery system 100 configured as a peripheral deploy system on the top of each of the images (which may be provided in accordance with one or more embodiments) and a conventional deploy system on the bottom of each of the images.

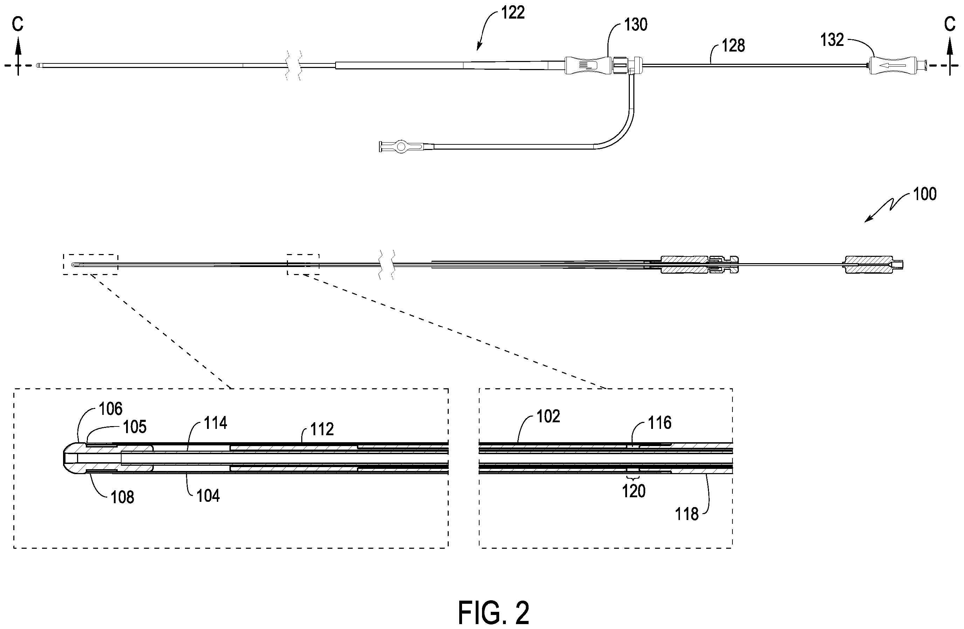

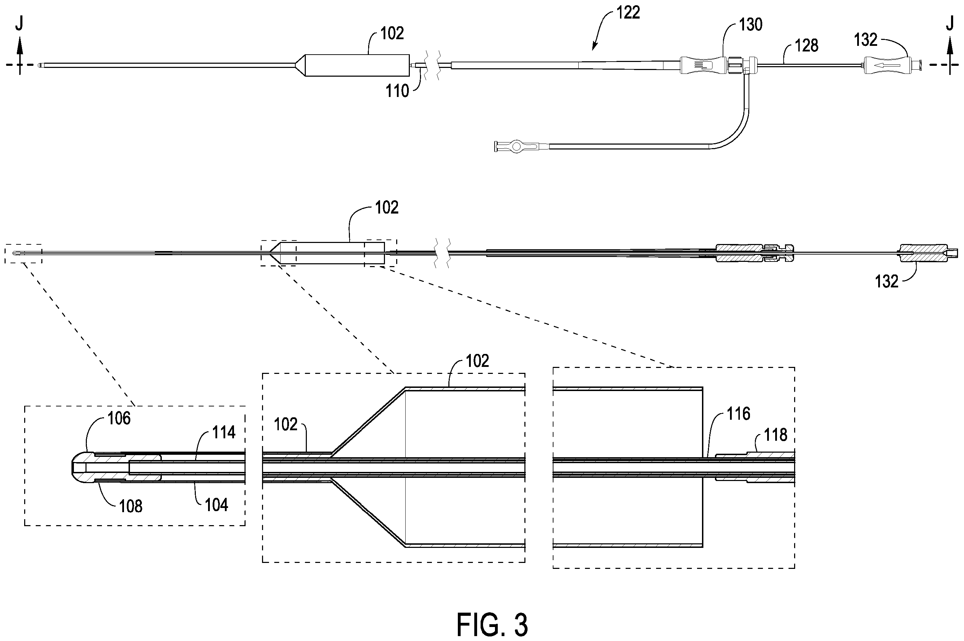

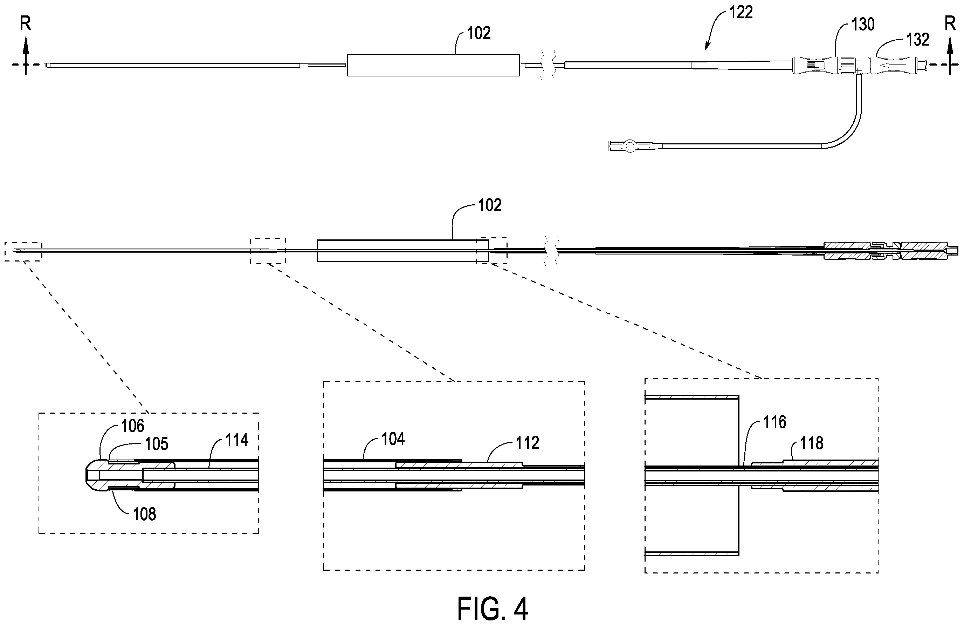

Figure 1 illustrates the stent delivery system 100 (also referred to as a stent deployment system) in three different stages or states of deployment, including a collapsed or a non-deployed stage or state (shown in more detail in Figure 2), a partially deployed stage or state (shown in more detail in Figure 3) and in a fully deployed stage or state (shown in more detail in Figure 4). As can be seen, the stent delivery system 100 includes a nose cone 106 at an end thereof that has a shoulder region or engagement portion 105 formed by a crimp ring 108 to fixedly engage the nose cone 106 to the end of the outer stent cover 104. Thus, during deployment, the nose cone 106, which is also coupled to an end of an inner shaft 114 (an inner elongate shaft is illustrated), which when moved (e.g., advanced from right to left in Figures 2-4), causes the outer stent cover 104 to move in a proximal to distal direction to allow the stent 102 contained in a collapsed configuration therein, to be deployed.

As can be seen in the Figures, a stent stop 112 (e.g., a ring, a band, a step, a bushing, or a sleeve) is provided at a distal end of the stent 102 to position the stent 102 within the outer stent cover 104. It should be appreciated that the position of the stent stop 102 in the outer stent cover 104 may be changed as desired or needed, for example, to be located closer or farther away from the distal end of the stent delivery system 100 (the nose cone 106 end). Thus, the stent stop 112 facilitates engaging and/or containing the stent 102 in a desired or predetermined position within the outer stent cover 104.

The stent delivery system 100 further includes the inner shaft 114 that is coupled to the nose cone 106, which is coupled to the outer stent cover 104, such that during deployment of the stent 102, these components moved together. Thus, the inner shaft 114 is positioned radially inward of the outer stent cover 104 such that a concentric arrangement of the elements is provided. The inner shaft 114 extends longitudinally within the outer stent cover 104 in this concentric arrangement. It should be noted that a portion of the system extends beyond a distal end of a body portion 122 of the stent delivery 100 (as can be seen at the top of Figure 2). The inner shaft 114 may include a lumen extending between the proximal and distal portions thereon, with the lumen configured to slidably receive a guide wire.

The stent delivery system 100 further includes a midshaft 116 (or middle shaft) provided between the stent 102 and an outer shaft 118. The outer shaft 118 extends from a portion within the proximal end of the outer stent cover 104 to outside the outer stent cover 104. Thus, in various embodiments, the outer shaft 118 has a reduced diameter portion 120 that extends into the outer stent cover 104 and abuts the midshaft 116. The remainder of the outer shaft 118 that is not contained within the outer stent cover 104 has a diameter that is the same as or approximately the same as the outer stent cover 104.

As can be seen at the top of Figure 2, in the non-deployed stage or state, the stent 102 is not visible and contained within the stent delivery system 100. In this state, the stent 102 may be delivered to a position of interest within the anatomy while in the collapsed stated and contained within the stent delivery system.

As illustrated in Figure 3, the stent 102 is shown in a partially deployed stage or state, which is about 50% deployed in this Figure. As can be seen, the outer stent cover 104 is advanced such that the stent 102 is deployed from a peripheral end of the outer stent cover 104. In particular, as a force is applied by the stent delivery system (the force may be applied automatically, semi-automatically or manually), which may be applied using different techniques, including techniques in the technological art of advancing a stent from a delivery system, such that the outer stent cover 104 is moved as the nose cone 106 portion is advanced such that the stent 102 expands from the proximal end of the outer stent cover 104. As can be seen, the stent 102 expands to the distal side of the outer shaft 118, such that the stent 102 expands about and along the midshaft 116 between the proximal end of the outer stent cover 104 and the distal end of the outer shaft 118. The distal end assembly portion of the stent delivery system 100, including the nose cone 106 that is coupled with the outer stent cover 104, is moved to allow the proximal to distal direction deployment of the stent.

Thus, the outer shaft 118 is removed from within the outer stent cover 104 to allow the stent 102 to expand in a proximal to distal direction. As should be appreciated, the outer stent cover 104 is being moved relative to the stent in the proximal to distal direction (forward) instead of the outer stent cover 104 being pulled backward to expose and expand the stent 102 in the distal to proximal direction (see also Figure 5 illustrating the differences in deployment of the stent 102). Improved accuracy of placement of the stent 102 (e.g., peripheral venous stent), such as in the venous system of an individual is provided with the deployment using the stent delivery system 100.

As illustrated in Figure 4, the stent 102 is shown in a fully deployed stage or state. As can be seen, the outer stent cover 104 is advanced such that the stent 102 is fully exposed and deployed from a peripheral end of the outer stent cover 104. In this fully deployed stage or state, the stent stop 112 has moved away from the stent 102 and is spaced apart therefrom. However, in the illustrated embodiment, the relative position of the midshaft 116 and outer shaft 118 relative to the proximal end of the stent 102 has not changed from the partially deployed stage or state shown in Figure 3. Improved positioning and maintaining the placement of the stent 102, including the proximal end of the stent 102 can be provided.

As can be seen in Figures 2-4, a tube 128 (e.g., a hypotube) is moved to cause the outer stent cover 104 to advanced such that the stent 102 is exposed. In particular, the tube 128 is advanced toward a hub 130 (which in some embodiments is an end hub of the outer shaft 118) by applying a force (e.g., axial force) to a hub 132 (e.g., hypotube hub) until the hub 132 abuts the hub 130. At this point, the stent 102 is fully deployed from the outer stent cover 104.

Figure 5 illustrates the deployment of the stent 102 (e.g., a 16x120 mm stent) using the stent delivery system 100 (the upper portion in each of steps (1)-(4)) in the proximal to distal direction (forward) instead of the distal to proximal direction using a stent delivery system 200 (a portion of which is shown in the lower portion of each of steps (1)-(4)). In Figure 5, step (1) illustrates a packaged stent delivery system 100 (in a non-deployed stage or state), steps (2) and (3) illustrate states of partial deployment of the stent 102 and step (4) illustrates full or complete deployment of the stent 102.

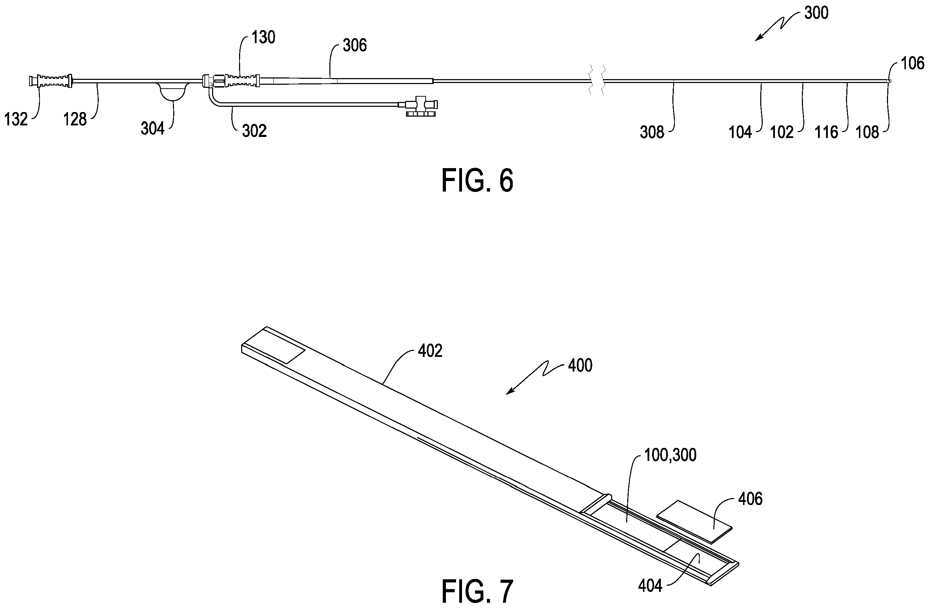

A stent delivery system 300 is shown in Figure 6. It should be noted that like numerals represent like parts in the various Figures. The delivery system 300 operates like the delivery system 100 to deliver the stent 102 in the proximal to distal direction. In operation, the front or distal end of the stent delivery system 300 is moved to advance the outer stent cover 104 to expose the stent 102 contained therein. For example, in one more embodiments, the nose cone 106 is coupled at the end of the inner shaft 114 such that a tipped inner shaft is formed. Additionally, a strain relief 306 is provided at an end of the outer shaft 118.

The stent delivery system 300 includes a valve 302, such as a hemostasis valve with a side port that allows purging of the system. For example, in some embodiments, the valve 302 allows for saline to be flushed through the system to purge the lumen therein.

Additionally, the stent delivery system 300 includes a clip 304, which in the illustrated embodiment is a safety clip that is coupled over the tube 128 to prevent movement thereof. The clip 304 may be removably coupled to the tube 128 using different techniques (e.g., snap fit) to act as a physical stop and, for example, prevent inadvertent operation or deployment of the stent 102. Thus, when operation of the stent delivery system 300 is desired, the clip 304 is removed from the tube 128.

Variations and modifications are contemplated. For example, in some embodiments, a marker 308 (e.g., a radio-opaque marker band) is provided at a predetermined location of the stent delivery system 300, such as around a circumference of the outer stent cover 104. The marker 308 may be used to facilitate locating that portion of the stent delivery system 300 when being used to facilitate placement thereof for subsequent deployment of the stent 102 such as by using x-ray imaging (e.g., fluoroscopy).

A delivery system 400 (or transport system) such as shown in Figure 7 may be provided for delivery of the stent delivery system 100 or 300, or other stent delivery system. The delivery system 400 provides for protecting the stent delivery system 100 or 300 and maintaining the stent delivery system 100 or 300 sterile during transport.

The delivery system 400 includes a portion for receiving therein the stent delivery system 100 or 300, such as within a pouch and box 402 (e.g., a shelf box). Additionally, a portion of the delivery system 400 includes space 404 for receiving therein instruction 406, such as operating instructions, instructions for use, or the like. It should be noted that multiple delivery systems 400 may be bundled together in a box for transport.

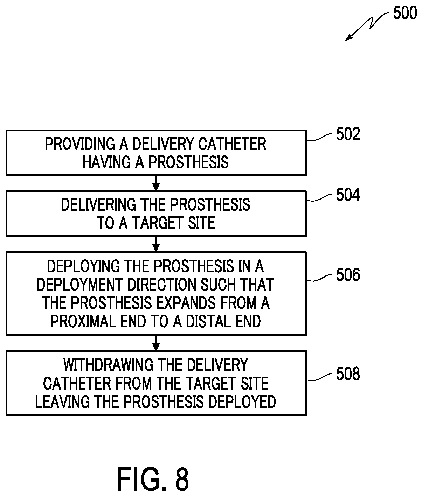

A process for stent deployment is illustrated by the method 500 shown in Figure 8. It should be noted that the method 500 may be employed or performed in connection with one or more embodiments disclosed herein, such as with the stent delivery system 100 or 300. One or more steps in the method 500 may be removed, additional steps may be added, and the steps may be performed in a different order than shown. Additionally, one or more the steps may be performed simultaneously, concurrently or sequentially.

The method 500 in some embodiments is a peripheral deployment method for deploying a prosthesis (e.g., the stent 102) at a treatment site in a patient. The method 500 includes at 502 providing a delivery catheter (e.g., the stent delivery system 100 or 300) comprising the prosthesis having a proximal end and a distal end. The prosthesis is in a collapsed configuration and disposed on the delivery catheter such as described herein. The method 500 includes delivering the prosthesis to a target site (e.g., a target treatment site) at 104. For example, the prosthesis may be moved to a desired location within the venous system of a patient.

The method 500 further includes deploying the prosthesis at 506, wherein the deployment direction causes the prosthesis to radially expand from the proximal end thereof to the distal end thereof as described herein. During the deployment of the prosthesis, a constraint (e.g., the outer stent cover 104) is removed from the prosthesis, thereby permitting the prosthesis to radially expand in the peripheral deployment direction. As the constraint is removed, the prosthesis radially expands from the collapsed configuration to the expanded configuration in the peripheral deployment direction so that the expanded prosthesis, for example, engages tissue at the target. The method 500 includes withdrawing the delivery catheter at 508, such as removing the delivery catheter from the patient and leaving the prosthesis deployed at the target site.

In some aspects, a method, which may form part of the method 500, includes configuring a delivery subsystem (embodied as, being part of or in combination with the delivery catheter) to have a nose tip coupled with a sheath such that the nose tip and sheath are movable together, wherein the sheath is configured to receive therein a stent for deployment. The stent surrounds an inner shaft of the delivery subsystem. The method may include configuring an actuating subsystem (which may be all or part of the stent delivery system 100 or 300) to allow advancement of the sheath along the inner shaft from a proximal to distal direction to deliver the stent in a reverse deploy direction as described herein. For example, the method provides for advancing a sheath of a stent delivery system from a proximal end to a distal end to deliver a stent contained within the sheath.

A stent delivery system in some aspects includes an inner elongate shaft having a proximal portion and a distal portion, and a radially expandable prosthesis disposed over the inner elongate shaft, the prosthesis having a radially collapsed configuration and a radially expanded configuration, wherein in the collapsed configuration the prosthesis is adapted to be delivered through a patient's vasculature, and in the expanded configuration the prosthesis engages a vessel wall. The stent delivery system also includes an outer elongate shaft having a proximal portion and a distal portion and a sheath or cover having a proximal portion and a distal portion, wherein the sheath or cover is disposed over the radially expandable prosthesis. In operation, distal advancement of the inner shaft advances the sheath or cover distally, thereby allowing the prosthesis to radially expand from a proximal end thereof to a distal end thereof.

In some aspects, a stent delivery system includes a delivery subsystem having a nose tip coupled with a sheath such that the nose tip and sheath are movable together. The sheath is configured to receive therein a stent for deployment, with the stent surrounding an inner shaft of the delivery subsystem. In this aspect, an actuating subsystem is configured to advance the sheath along the inner shaft from a proximal to distal direction to deliver the stent in a reverse deploy direction. In some aspects, the delivery subsystem and actuating subsystem are configured to deliver the stent in only a reverse deploy direction

It should be noted that a peripheral deploy stent system in accordance with one or more embodiments may be used in conjunction with a conventional stent delivery system, such as to implant multiple stents. For example, a "bracketing" technique as known in the art may be utilized by the physician. Using this technique, the physician would begin by stenting both ends of the targeted treatment zone, then stent the region therebetween. This technique allows the physician to accurately place and position the more critical end of the stent implant on either end of the targeted treatment zone.

For example, in the case of iliofemoral stenting (assuming femoral access), the physician could use a conventional stent delivery system for stent placement of the distal stent (relative to the delivery system) at the confluence between the iliac and IVC. A peripheral deploy system as described herein then could be used to place the stent at the most peripheral end of the treatment zone. The more accurate placement of the proximal most stent (relative to the delivery system) would ensure stent coverage over the native vessel and would prevent jailing of the Profunda vein. Conventional stent delivery system(s) then could be used to stent the region between the central and peripheral stents. It should be noted that the various embodiments also may be used alone (i.e., not in combination with conventional deploy systems).

It also should be noted that in various embodiments, a central direction generally refers to a direction towards the IVC and a peripheral direction refers to a direction away from the IVC. However, as should be appreciated, regardless of the direction of the deployment relative to anatomical structures, the stent in various embodiments is deployed from a proximal to distal end as the sheath is pushed further along the lumen with the nose cone or tip that is fixedly coupled thereto, such that the nose cone or tip and sheath move together. This configuration is in contrast to a conventional delivery system such as shown in Figure 5 (bottom system in each of the images), wherein the tip of the delivery system remains in place as the sheath is withdrawn therefrom (e.g., no change in length of the overall system).

Thus, in operation, using a peripheral or reverse deploy system in accordance with various embodiments, the sheath is not pulled away from the tip to expose the stent, but instead, the sheath is pushed with the nose cone or tip to expose the stent in the opposite direction of deployment from a conventional deployment system as can be seen in the Figures, such as Figure 5. In various embodiments, the sheath and inner member (in some embodiments) are coupled with the nose cone or tip to move together. It should be noted that the coupling of the various embodiments may be provided to fixedly secure the components together so that the components do not separate during operation. In some embodiments, the nose cone or tip and sheath may be formed as a single integrally piece formed in a unitary design. However, in other embodiments, these components may be coupled together with an adhesive or other suitable coupling substance or member (e.g., a bracket).

As should be appreciated, various embodiments are configured as endoluminal delivery systems for prostheses such as stents, or other implantable structures. Thus, the delivery systems described herein may be used for placement of a stent in the arterial system, the venous system, or any other portion of the body.

It should be noted that various embodiments may be used in connection with other systems that facilitate identifying the particular location within the body at which one or more stents are to be placed.

This written description uses examples to disclose the various embodiments, including the best mode, and also to enable any person skilled in the art to practice the various embodiments, including making and using any devices or systems and performing any incorporated methods. The patentable scope of the various embodiments is defined by the claims, and may include other examples that occur to those skilled in the art. Such other examples are intended to be within the scope of the claims if the examples have structural elements that do not differ from the literal language of the claims, or if the examples include equivalent structural elements with insubstantial differences from the literal languages of the claims.

It is to be understood that the above description is intended to be illustrative, and not restrictive. For example, the above-described embodiments (and/or aspects thereof) may be used in combination with each other. In addition, many modifications may be made to adapt a particular situation or material to the teachings of the invention without departing from its scope. Dimensions, types of materials, orientations of the various components, and the number and positions of the various components described herein are intended to define parameters of certain embodiments, and are by no means limiting and are merely exemplary embodiments. Many other embodiments and modifications within the scope of the claims will be apparent to those of skill in the art upon reviewing the above description. The scope of the invention should, therefore, be determined with reference to the appended claims, along with the full scope of equivalents to which such claims are entitled. In the appended claims, the terms "including" and "in which" are used as the plain-English equivalents of the respective terms "comprising" and "wherein." Moreover, in the following claims, the terms "first," "second," and "third," etc. are used merely as labels, and are not intended to impose numerical requirements on their objects.

## Claims

1. A stent delivery system (100) comprising:
an inner elongate shaft (114) having a proximal portion and a distal portion;
a radially expandable prosthesis (102) disposed over the inner elongate shaft (114), the prosthesis (102) having a radially collapsed configuration and a radially expanded configuration, wherein in the collapsed configuration the prosthesis (102) is adapted to be delivered through a vasculature, and in the expanded configuration the prosthesis (102) engages a vessel wall;
an outer elongate shaft (118) having a proximal portion and a distal portion;
a sheath (104) having a proximal portion and a distal portion, the sheath (104) disposed over the radially expandable prosthesis (102);
a first hub (130) coupled to the outer shaft (118);
a second hub (132) disposed proximally of the first hub (130); and
a hypotube (128) coupled to the second hub (132) and extending distally toward the first hub (130),
wherein distal advancement of the inner shaft (114) advances the sheath (104) distally, thereby allowing the prosthesis (102) to radially expand from a proximal end thereof to a distal end thereof.

2. The stent delivery system of claim 1, wherein the radially expandable prosthesis (102) is a stent, and further comprising (i) a delivery subsystem having a nose tip (106) coupled with the sheath (104) such that the nose tip (106) and sheath (104) are movable together, the sheath (104) configured to receive therein the stent for deployment, the stent surrounding the inner elongate shaft (114) and (ii) an actuating subsystem configured to advance the sheath (104) long the inner elongate shaft (114) from a proximal to distal direction to deliver the stent in a reverse deploy direction.

3. The stent delivery system of claim 2, wherein the delivery subsystem and actuating subsystem are configured to deliver the stent in only a reverse deploy direction.

4. The stent delivery system of claim 1, wherein the inner elongate shaft (114) comprises a lumen extending between the proximal and distal portions, the lumen configured to slidably receive a guide wire.

5. The stent delivery system of claim 1, wherein the prosthesis (102) comprises a stent.

6. The stent delivery system of claim 5, wherein the stent comprises a self-expanding stent.

7. The stent delivery system of claim 5, wherein the stent comprises a nickel titanium alloy.

8. The stent delivery system of claim 1, wherein the outer shaft (118) comprises a lumen extending between the proximal and distal portions thereof and the sheath (104) has a length greater than or equal to a length of the radially expandable prosthesis (102), the sheath (104) configured to constrain the prosthesis (102) along substantially the entire length thereof.

9. The stent delivery system of claim 1, further comprising a midshaft (116) concentric with the inner and the outer shafts (114, 118), and disposed therebetween.

10. The stent delivery system of claim 9, wherein the midshaft (116) comprises a distal stent stop (112) disposed distally of a distal end of the prosthesis (102), wherein the distal stent stop (112) prevents distal movement of the prosthesis (102).

11. The stent delivery system of claim 10, wherein the distal stent stop (112) comprises one or more of ring, a band, a step, a bushing, or a sleeve, that prevents distal movement of the prosthesis (102).

## Patentansprüche

1. Stent-Zuführungssystem (100), das aufweist:
einen inneren länglichen Schaft (114) mit einem proximalen Abschnitt und einem distalen Abschnitt;
eine radial expandierbare Prothese (102), die über dem inneren länglichen Schaft (114) angeordnet ist, wobei die Prothese (102) eine radial kollabierte Konfiguration und eine radial expandierte Konfiguration aufweist, wobei die Prothese (102) in der kollabierten Konfiguration eingerichtet ist, durch ein Gefäßsystem zugeführt zu werden, und in der expandierten Konfiguration die Prothese (102) mit einer Gefäßwand in Eingriff tritt;
einen äußeren länglichen Schaft (118) mit einem proximalen Abschnitt und einem distalen Abschnitt;
eine Hülle (104) mit einem proximalen Abschnitt und einem distalen Abschnitt, wobei die Hülle (104) über der radial expandierbaren Prothese (102) angeordnet ist;
ein erstes Ansatzstück (130), das mit dem äußeren Schaft (118) gekoppelt ist;
ein zweites Ansatzstück (132), das proximal vom ersten Ansatzstück (130) angeordnet ist; und
ein Hypotube (128), der mit dem zweite Ansatzstück (132) gekoppelt ist und sich distal zum ersten Ansatzstück (130) erstreckt,
wobei das distale Vorschieben des inneren Schafts (114) die Hülle (104) distal vorschiebt,
wodurch es ermöglicht wird, dass die Prothese (102) von deren proximalen Ende zu deren distalen Ende radial expandiert.

2. Stent-Zuführungssystem nach Anspruch 1, wobei die radial expandierbare Prothese (102) ein Stent ist und ferner aufweist: (i) ein Zuführungsteilsystem mit einer Nasenspitze (106), die mit der Hülle (104) gekoppelt ist, so dass die Nasenspitze (106) und die Hülle (104) zusammen beweglich sind, wobei die Hülle (104) konfiguriert ist, darin den Stent zur Entfaltung aufzunehmen, wobei der Stent den inneren länglichen Schaft (114) umgibt, und (ii) ein Betätigungsteilsystem, das konfiguriert ist, die Hülle (104) längs des inneren länglichen Schafts (114) von einer proximalen zu einer distalen Richtung vorzuschieben, um den Stent in einer umgekehrten Entfaltungsrichtung zuzuführen.

3. Stent-Zuführungssystem nach Anspruch 2, wobei das Zuführungsteilsystem und das Betätigungsteilsystem konfiguriert sind, den Stent nur in einer umgekehrten Entfaltungsrichtung zuzuführen.

4. Stent-Zuführungssystem nach Anspruch 1, wobei der innere längliche Schaft (114) ein Lumen aufweist, das sich zwischen dem proximalen und dem distalen Abschnitt erstreckt, wobei das Lumen konfiguriert ist, verschiebbar einen Führungsdraht aufzunehmen.

5. Stent-Zuführungssystem nach Anspruch 1, wobei die Prothese (102) einen Stent aufweist.

6. Stent-Zuführungssystem nach Anspruch 5, wobei der Stent einen selbstexpandierenden Stent aufweist.

7. Stent-Zuführungssystem nach Anspruch 5, wobei der Stent eine Nickel-Titan-Legierung aufweist.

8. Stent-Zuführungssystem nach Anspruch 1, wobei der äußere Schaft (118) ein Lumen aufweist, das sich zwischen dessen proximalen und distalen Abschnitt erstreckt, und die Hülle (104) eine Länge aufweist, die größer oder gleich einer Länge der radial expandierbaren Prothese (102) ist, wobei die Hülle (104) konfiguriert ist, die Prothese (102) im Wesentlichen über ihre gesamte Länge einzuschränken.

9. Stent-Zuführungssystem nach Anspruch 1, das ferner einen Mittelschaft (116) aufweist, der mit dem inneren und dem äußeren Schaft (114, 118) konzentrisch ist und dazwischen angeordnet ist.

10. Stent-Zuführungssystem nach Anspruch 9, wobei der Mittelschaft (116) einen distalen Stent-Anschlag (112) aufweist, der distal von einem distalen Ende der Prothese (102) angeordnet ist, wobei der distale Stent-Anschlag (112) eine distale Bewegung der Prothese (102) verhindert.

11. Stent-Zuführungssystem nach Anspruch 10, wobei der distale Stent-Anschlag (112) einen Ring und/oder ein Band und/oder eine Stufe und/oder eine Buchse und/oder eine Hülse aufweist, die eine distale Bewegung der Prothese (102) verhindern.

## Revendications

1. Système de pose de stent (100) comprenant:
une tige allongée interne (114) ayant une partie proximale et une partie distale;
une prothèse radialement expansible (102) disposée au-dessus de la tige allongée interne (114), la prothèse (102) ayant une configuration radialement affaissée et une configuration radialement expansée, où dans la configuration affaissée la prothèse (102) est adaptée pour être posée par le biais d'un système vasculaire, et dans la configuration expansée la prothèse (102) vient en contact avec une paroi de vaisseau;
une tige allongée externe (118) ayant une partie proximale et une partie distale;
une gaine (104) ayant une partie proximale et une partie distale, la gaine (104) disposée au-dessus de la prothèse radialement expansible (102);
un premier embout (130) couplé à la tige externe (118);
un second embout (132) disposé de manière proximale par rapport au premier embout (130); et
un hypotube (128) couplé au second embout (132) et s'étendant de manière distale vers le premier embout (130),
où l'avancement distal de la tige interne (114) fait avancer la gaine (104) de manière distale, permettant ainsi à la prothèse (102) de s'expanser radialement depuis une extrémité proximale de celle-ci jusqu'à une extrémité distale de celle-ci.

2. Système de pose de stent selon la revendication 1, où la prothèse radialement expansible (102) est un stent, et comprenant en outre (i) un sous-système de pose ayant une pointe de nez (106) couplée à la gaine (104) de telle sorte que la pointe de nez (106) et la gaine (104) sont mobiles ensemble, la gaine (104) configurée pour y recevoir le stent pour le déploiement, le stent entourant la tige allongée interne (114) et (ii) un sous-système d'actionnement configuré pour faire avancer la gaine (104) le long de la tige allongée interne (114) d'une direction proximale à distale pour poser le stent dans une direction de déploiement inverse.

3. Système de pose de stent selon la revendication 2, où le sous-système de pose et le sous-système d'actionnement sont configurés pour poser le stent dans une direction de déploiement inverse seulement.

4. Système de pose de stent selon la revendication 1, où la tige allongée interne (114) comprend une lumière s'étendant entre les parties proximale et distale, la lumière configurée pour recevoir de manière coulissante un fil de guidage.

5. Système de pose de stent selon la revendication 1, où la prothèse (102) comprend un stent.

6. Système de pose de stent selon la revendication 5, où le stent comprend un stent à auto-expansion.

7. Système de pose de stent selon la revendication 5, où le stent comprend un alliage de nickel et de titane.

8. Système de pose de stent selon la revendication 1, où la tige externe (118) comprend une lumière s'étendant entre les parties proximale et distale de celle-ci et la gaine (104) a une longueur supérieure ou égale à une longueur de la prothèse radialement expansible (102), la gaine (104) configurée pour contraindre la prothèse (102) sur sensiblement toute sa longueur.

9. Système de pose de stent selon la revendication 1, comprenant en outre une tige intermédiaire (116) concentrique avec les tiges interne et externe (114, 118), et disposée entre elles.

10. Système de pose de stent selon la revendication 9, où la tige intermédiaire (116) comprend un arrêt de stent distal (112) disposé de manière distale par rapport à une extrémité distale de la prothèse (102), où l'arrêt de stent distal (112) empêche le mouvement distal de la prothèse (102).

11. Système de pose de stent selon la revendication 10, où l'arrêt de stent distal (112) comprend un ou plusieurs d'un anneau, d'une bande, d'un gradin, d'une douille ou d'un manchon, qui empêche le mouvement distal de la prothèse (102).
